(19) Europäisches Patentamt / European Patent Office / Office européen des brevets

(11) **EP 0 888 780 B2**

(12) **NEW EUROPEAN PATENT SPECIFICATION**
After opposition procedure

(45) Date of publication and mention
of the opposition decision:
**19.08.2009 Bulletin 2009/34**

(45) Mention of the grant of the patent:
**08.10.2003 Bulletin 2003/41**

(21) Application number: **97912423.7**

(22) Date of filing: **12.11.1997**

(51) Int Cl.:
**A61L 2/18** (2006.01)

(86) International application number:
**PCT/JP1997/004108**

(87) International publication number:
**WO 1998/020912 (22.05.1998 Gazette 1998/20)**

(54) **TREATMENT COMPOSITION FOR CONTACT LENSES AND METHOD FOR TREATING CONTACT LENSES WITH THE SAME**

ZUSAMMENSETZUNG ZUR BEHANDLUNG VON KONTAKTLINSEN UND METHODE ZUR ANWENDUNG DERSELBEN

COMPOSITION DE TRAITEMENT POUR LENTILLES DE CONTACT ET PROCEDE DE TRAITEMENT DE LENTILLES DE CONTACT AU MOYEN DE LADITE COMPOSITION

(84) Designated Contracting States:
**DE FR GB**

(30) Priority: **13.11.1996 JP 30163996**
**04.08.1997 JP 20934597**

(43) Date of publication of application:
**07.01.1999 Bulletin 1999/01**

(73) Proprietor: **Menicon Co., Ltd.**
**Nagoya-shi, Aichi 460-0006 (JP)**

(72) Inventors:
• **NAKAGAWA, Akira,**
**Tomey Technology Corporation**
**Nagoya-shi,**
**Aichi 451 (JP)**
• **NIWA, Yoshiko,**
**Tomey Technology Corporation**
**Nagoya-shi,**
**Aichi 451 (JP)**
• **KOZAWA, Takako,**
**Tomey Technology Corporation**
**Nagoya-shi,**
**Aichi 451 (JP)**
• **TSUBOI, Keiko,**
**Tomey Technology Corporation**
**Nagoya-shi,**
**Aichi 451 (JP)**
• **YOSHIHARA, Kyoko,**
**Tomey Technology Corporation**
**Nagoya-shi,**
**Aichi 451 (JP)**

(74) Representative: **Paget, Hugh Charles Edward et al**
**Mewburn Ellis LLP**
**33 Gutter Lane**
**London**
**EC2V 8AS (GB)**

(56) References cited:
EP-A- 0 001 888 WO- -96//02624
JP-A- 7 146 454 JP-A- 8 029 744
JP-A- 8 271 841 US- - 4 581 332
US- - 5 422 073

EP 0 888 780 B2

**Description**

Technical Field

The present invention relates to a treating composition for a contact lens and a method of treating the contact lens using the same. More specifically described, the invention relates to a composition which prevents the contact lens from being stained, especially with a proteinaceous substance, and a method of preventing the contact lens from being stained by using the composition. Further, the present invention is concerned with a liquid cleaning composition for a contact lens and a method of cleaning the contact lens using the same. The present invention particularly relates to an improvement of the liquid cleaning composition which includes a protease that is capable of decomposing and removing a proteinaceous substance from the contact lens, and a method of cleaning the contact lens using such a liquid cleaning composition.

Background Art

Contact lenses are classified into water-swellable lenses and non-water-swellable lenses. These contact lenses are soiled with stains or deposits such as protein, lipid, and inorganic substances during wearing of the contact lenses on the eyes, which deposits are derived from tear fluid and lipid of the eyes. These deposits adhering to the contact lenses deteriorate the wearing comfort of the contact lenses as felt by the lens wearer, lower the eyesight of the lens wearer, and cause various other troubles with the eyes such as hyperemia of the conjunctive. In view of this, it is required to regularly clean the contact lenses so as to remove the deposits therefrom for continuous wearing of the contact lenses. In particular, bacteria tend to adhere to, or proliferate on the water-swellable contact lenses. Therefore, it is required to disinfect the water-swellable contact lenses in addition to carrying out the cleaning treatment.

Various kinds of deposits as described above are removed from the contact lenses by various cleaning methods suitably selected to deal with the respective deposits. For instance, the protein deposits accumulated on the contact lens surfaces are removed therefrom by immersing the contact lens in a solution which includes a protease, so that the protein deposits are decomposed by the protease. The lipid deposits are removed from the contact lens by immersing the contact lens in a solution which includes a surface active agent or by rubbing the contact lens with fingers using the solution. The inorganic deposits are removed from the contact lens by immersing the lens in a solution which includes a metal chelating agent, so as to block metal ions such as calcium ions in the inorganic deposits.

Conventionally, a thermal disinfecting method is effected for disinfecting the contact lens, wherein the contact lens accommodated in a contact lens case is boiled by using a boiling device exclusively used for disinfecting the contact lenses. In recent years, in addition to the thermal disinfecting method, a chemical disinfecting method is effected for disinfecting the contact lens, wherein the contact lens is immersed in a liquid agent which contains a disinfectant.

Since the protease used for removing the protein deposits from the contact lens is unstable in a dilute solution, the protease is provided in a tablet form or in a concentrated liquid form. Accordingly, when the contact lens is cleaned for removal of the protein deposits, the protease in the tablet or concentrated liquid form needs to be dissolved or diluted, making the cleaning treatment of the contact lens cumbersome. Even if the contact lens is regularly cleaned for removal of the protein deposits, it is quite difficult to completely remove the protein deposits adhering to the contact lens. Therefore, the protein deposits are gradually accumulated on the contact lens over a long period of wearing of the contact lens. In this case, the wearing comfort of the contact lens is inevitably deteriorated with an increase in the wearing time of the contact lens.

The protease used to decompose and remove the protein deposits which are accumulated on the contact lens is unstable in a solution, and its activity is gradually lowered in the solution. In view of this, the protease is practically used in a solid form such as tablet, granule or powder that contains the protease as a main component. The protease in a solid form as described above is dissolved as needed in purified water, for instance, so as to provide a cleaning liquid for cleaning the contact lens. In this method, however, the solid protease must be dissolved each time the contact lens is cleaned, undesirably making the cleaning treatment cumbersome. Moreover, the enzyme activity of the protease is gradually lowered after the protease is dissolved in a solution. Thus, it is quite difficult to include the protease in a storing liquid for storing the contact lens.

In view of the above, various liquid-type cleaning agents are conventionally proposed and are commercially available, wherein the protease is stabilized in a solution. For instance, JP-A-2-168224 discloses a contact lens cleaning liquid in which the protease and an organic solvent which is miscible with water, such as glycerin, propylene glycol or polyethylene glycol are mixed in water. This cleaning liquid is diluted with an aqueous medium, so as to provide a dilution in which the contact lens is immersed for decomposing and removing the protein deposits adhering thereto. JP-A-4-93919 and JP-A-4-143718 disclose a liquid-type cleaning agent which includes a specific protease, and a polyhydric alcohol such as ethylene glycol, propylene glycol or glycerin in an amount of not smaller than 20 wt.%. JP-A-4-161921 discloses a cleaning agent which includes a saccharide such as a monosaccharide or a disaccharide as a stabilizing component.

In the proposed cleaning liquids which contain the protease, however, the protease is not stabilized to a sufficient extent, and its activity is gradually lowered with a lapse of time. Accordingly, the proposed cleaning liquids suffer from reduction in its cleaning effect for the contact lens while the liquids are stored for a long period of time. Thus, it is desired to keep the protease in the solution with sufficiently high stability. Even if the contact lens is cleaned by using the proposed cleaning liquids for removal of the protein deposits on a regular basis, it is quite difficult to completely remove the protein deposits adhering to the contact lens. Accordingly, the protein deposits are gradually accumulated on the contact lenses over a long period of wearing of the contact lens, and the wearing comfort is inevitably deteriorated with an increase in the wearing time of the contact lens. In addition, the protein deposits which has been removed by the protease from the contact lens during the cleaning treatment (,in other words, components of the protein deposits decomposed by the protease) tend to adhere back to the contact lens, undesirably soiling the contact lens.

In an attempt to avoid the deterioration of the wearing comfort of the contact lens due to the accumulation of the deposits thereon, it has been proposed to introduce a so-called "frequent replacement system" in wihch the contact lenses are replaced with new ones on a regular basis, and disposable contact lenses are commercially available. According to these measures, the contact lenses are replaced with new ones before the protein deposits are accumulated thereon, although the wearing period of the contact lenses is reduced. Therefore, these measures can always assure the lens user of a good wearing comfort with a simple treatment of the contact lenses. These measures, however, inevitably push up the cost of maintenance of the contact lenses, as compared with that of the conventional contact lenses, leading to an increase in the economical burden on the user.

Disclosure of the Invention

The present invention was developed in the light of the above situations. It is therefore a first object of the present invention to provide a treatment of a contact lens with a composition which is safe to a living body and which prevents the contact lens from being stained, especially with a proteinaceous substance. It is a second object of the invention to provide a method of treating a contact lens using such a treating composition for preventing the contact lens from being stained. It is a third object of the invention to provide a liquid cleaning composition for a contact lens which assures easy handling and which effectively minimizes or prevents the staining of the contact lens, especially with the proteinaceous substance so as to exhibit a high cleaning effect while assuring a high stability in a liquid form. It is a fourth object of the invention to provide a method of quickly and effectively cleaning a contact lens using such a liquid cleaning composition.

The inventors of the present invention have made an extensive study in an effort to attain the above first and second objects, and found that 2-amino-2-methyl-1,3-propanediol and 2-amino-2-methyl-l-propanol which are conventionally used as a neutralizer or an emulsifier, or salts of these compounds are effective to minimize or prevent the staining of the contact lens, especially with the proteinaceous substance.

The above first object of the present invention may be attained according to the invention which provides use of a composition to treat a contact lens, as set out in claim 1.

Since the contact lens treating composition used according to the present invention includes the above-described compound(s) as the effective component for preventing the contact lens from being stained, the present contact lens treating composition is capable of effectively preventing the contact lens from being stained, especially with the proteinaceous substance while it is safe and has a low degree of toxicity to the eyes.

In the present contact lens treating composition, the above-described at least one compound is included in an amount of 0.01-3 wt.%. The treating composition which includes the at least one compound within the specified range advantageously provides an anti-staining effect or stain-preventing effect for preventing the contact lens from being stained, with its osmotic pressure being kept close to a physiological osmotic pressure.

The above second object of the present invention may be attained according to the invention which provides a method of preventing a contact lens from being stained, by using the above-described treating composition as set out in claim 5. Described more specifically, the present invention provides a method of preventing the contact lens from being stained, which is characterized by giving the contact lens an anti-staining property, the method comprising the steps of: preparing a treating solution which includes the contact lens treating composition as described above; and contacting the contact lens with the treating solution.

According to the treating method described above, the contact lens is not likely to be stained, especially with the proteinaceous substance, by simply contacting the contact lens with the treating solution that includes the contact lens treating composition described above, in a suitable manner for a predetermined period of time.

In one preferred form of the above method of preventing the staining of the contact lens, the contact lens is held in contact with the treating solution at a temperature of not lower than 80°C for not less than five minutes for simultaneously disinfecting the contact lens. In this arrangement, the contact lens can be simultaneously subjected to a stain-preventing treatment and a thermal disinfecting treatment.

In another preferred form of the method described above, the treating solution further includes an effective amount of disinfectant, and the contact lens is held in contact with the treating solution at room temperature for simultaneously. disinfecting the contact lens. In this arrangement, the contact lens can be simultaneously subjected to the stain-preventing treatment and the chemical disinfecting treatment.

In still another preferred form of the method described above, the treating solution further includes an effective amount of protease, and the contact lens is held in contact with the treating solution for simultaneously cleaning the contact lens. According to this arrangement, the contact lens can be simultaneously subjected to the stain-preventing treatment and a decomposition and cleaning treatment of the proteinaceous substance. Further, the contact lens is held in contact with the treating solution described above at a temperature of not lower than 80°C for not less than five minutes. In this arrangement, the contact lens can be subjected to the thermal disinfecting treatment in addition to the stain-preventing treatment and the decomposition and the cleaning treatment of the proteinaceous substance.

In yet another preferred form of the method described above, the treating solution further includes effective amounts of disinfectant and protease, and the contact lens is held in contact with the treating solution at room temperature. According to this arrangement, the contact lens can be subjected simultaneously to the stain-preventing treatment, the decomposition and cleaning treatment of the proteinaceous substance, and the chemical disinfecting treatment.

The inventors of the present invention have made an extensive study in an effort to attain the above third and fourth objects of the present invention, and found that the above-described compounds, namely, 2-amino-2-methyl-1,3-propanediol, 2-amino-2-methyl-1-propanol and salts thereof are considerably effective to stabilize the protease in the liquid cleaning composition if the compounds are present in the liquid cleaning composition with a high concentration, making it possible to keep the protease stable in the liquid cleaning composition for a long time period. The inventors further found that the above compounds effectively minimize or prevent the staining of the contact lens, especially with the proteinaceous substance when the liquid cleaning composition that contains the above compound(s) is diluted with a suitable aqueous diluting medium.

The above third object of the present invention may be attained according to the invention which provides a liquid cleaning composition for a contact lens, as set out in claim 2.

Since the liquid cleaning composition of the present invention includes, as a stabilizing component for stabilizing the protease, the above-described specific compound(s) (2-amino-2-methyl-1,3-propanediol, 2-amino-2-methyl-1-propanol and salts thereof); it is needless to say that the present liquid cleaning composition has a low degree of toxicity and is safe to the eyes. Moreover, the above-described specific compound(s) included in the present liquid cleaning composition are superior in its stabilizing effect for stabilizing the protease in the liquid cleaning composition, to conventionally used stabilizing agents such as glycerin and propylene glycol. Accordingly, the present liquid cleaning composition can be stored with high stability for a long period of time. When the contact lens is cleaned by using the present liquid cleaning composition, the contact lens is effectively prevented from being stained, especially with the proteinaceous substance. Accordingly, the present liquid cleaning composition advantageously inhibits the proteinaceous substance which was removed from the contact lens, i.e., the proteinaceous substance decomposed by the protease, from adhering back to the contact lens, whereby the contact lens is not stained with the decomposed proteinaceous substance. In addition, the contact lens which was cleaned by using the present liquid cleaning composition is not likely to be stained with the proteinaceous substance in subsequent wearing thereof, which substance is derived from the tear fluid or lipid in the eye.

In the liquid cleaning composition according to the present invention, the pH is adjusted to within a range of 5.5-7.5, in an attempt to further stabilize the protease therein.

The above fourth object of the present invention may be attained according to the present invention which provides a method of cleaning a contact lens, the method comprising the steps of: preparing a liquid cleaning composition which includes, in an aqueous medium, an effective amount of protease, and not less than 5 w/v% of at least one stabilizing component selected from the group consisting of 2-amino-2-methyl-1,3-propanediol, 2-amino-2-methyl-1-propanol, and salts thereof; diluting the liquid cleaning composition with an aqueous diluting medium so as to provide a dilution; and immersing in the dilution the contact lens which was worn on a human eye.

According to the method of cleaning a contact lens of the present invention, the contact lens is immersed in, and contacted with the dilution obtained by diluting the liquid cleaning composition described above, for a predetermined time period, so that the staining substance, particularly the proteinaceous substance on the contact lens can be effectively decomposed by the protease and removed from the contact lens. Further, the present liquid cleaning composition inhibits the decomposed proteinaceous substance from adhering back to the contact lens. The contact lens which was cleaned by using the present liquid cleaning composition is not likely to be stained with the proteinaceous substance in subsequent wearing thereof.

In one preferred form of the above method, the contact lens immersed in the dilution is boiled for at least five minutes, whereby the contact lens is simultaneously cleaned for removal of the proteinaceous substance and disinfected.

In another preferred form of the above method, the dilution further includes an effective amount of disinfectant, and the contact lens is immersed in the dilution at room temperature. According to this arrangement, the contact lens can be disinfected at the same time when it is cleaned for removal of the proteinaceous substance therefrom.

Best mode for carrying out the present invention

One aspect of the present invention is characterized by using 2-amino-2-methyl-1,3-propanediol, 2-amino-2-methyl-1-propanol or salts thereof as an effective component in the contact lens treating composition used for preventing the contact lens from being stained. Further, a method of preventing the staining of the contact lens is established using such a treating composition.

The above-described compounds, i.e., 2-amino-2-methyl-1,3-propanediol and 2-amino-2-methyl-1-propanol, as the effective component for preventing the staining of the contact lens are described in the Standard for cosmetic material, and are conventionally used as an emulsifier in a hand cream or a milky lotion, or as a neutralizer in a hair spray, for instance. The compounds are used in other fields than cosmetics, for example, as an emulsifier in a wax or cleaner for wiping a floor, or as a brightener in a urea resin or a melamine resin. At least one compound selected from among those compounds and salts thereof is used as the effective component in the present contact lens treating composition for preventing the contact lens from being stained.

The salts of 2-amino-2-methyl-1,3-propanediol and 2-amino-2-methyl-1-propanol are hydrochlorides, for example.

The above-described compound(s) exhibits an excellent effect of preventing the staining of the contact lens if the compound(s) is included in the treating composition generally with a concentration of 0.01-3 wt.%, preferably 0.05-2 wt.%, more preferably 0.2-1 wt.%. If the concentration of the compound(s) is lower than 0.01 wt.%, the treating composition does not provide the intended stain-preventing effect. On the other hand, if the concentration of the compound(s) is higher than 3 wt.%, the osmotic pressure of the treating composition deviates from the physiological osmotic pressure range, undesirably giving an adverse influence on the living body such as ocular irritation.

The above-described compounds, i.e. 2-amino-2-methyl-1,3-propanediol, 2-amino-2-methyl-1-propanol, and salts thereof are in a solid form at room temperature. For contacting the contact lens with the treating composition, the treating composition is preferably in a solution form, especially in an aqueous solution form. The above-described compound(s) in a solid form such as powder, tablet or granule is dissolved in purified water, physiological salt solution (or saline) or tap water, so as to provide the intended treating solution which includes the present contact lens treating composition. For obtaining the treating solution, the solid treating composition is dissolved by the user when needed. Alternatively, the treating composition which has been dissolved in purified water or physiological salt solution is provided in a container made of glass or plastic material.

The pH suitable for the present treating composition for preventing the staining of the contact lens is in a physiologically permissible range, namely, in a range of 5.0-9.0, preferably 6.0-8.0. The pH outside the above ranges may cause irritation or trouble to the eyes.

For keeping the pH within the above ranges to assure safety to the eyes in using the present contact lens treating composition, there is added a suitably selected pH regulator known in the art.

The compounds, i.e., 2-amino-2-methyl-1,3-propanediol and 2-amino-2-methyl-1-propanol, used as the effective component in the present contact lens treating composition for preventing the staining of the contact lens, are provided in a free state or in salts thereof such as hydrochlorides. When the compounds are present in a free state in the solution of the treating composition, the solution is alkaline. When the compounds are present in the form of hydrochlorides thereof in the solution, for instance, the solution is acidic. Accordingly, at least one acid or at least one alkali is added to the solution of the treating composition so as to adjust its pH in a neutral range. Examples of the acid to be added to the treating solution include hydrochloric acid, sulfuric acid, nitric acid, acetic acid, organic acid, boric acid and amino acid. It is preferable to employ hydrochloric acid or boric acid. Examples of the alkali include sodium hydroxide, potassium hydroxide and borax. Preferably used are sodium hydroxide and borax.

According to the method of preventing the contact lens from being stained, by using the thus prepared treating solution that contains the present treating composition, the contact lens is immersed in the treating solution, and then rinsed with water, for instance, whereby the contact lens is not likely to be stained, especially with the proteinaceous substance. In general, the contact lens is immersed in the treating solution at a temperature ranging from the room temperature to 110°C for a period of time ranging from five minutes to twenty-four hours.

To the treating agent or composition for preventing the staining of the contact lens prepared according to the present invention, the additives which are used in the conventional contact lens cleaning solutions may be suitably added, to thereby provide a disinfecting liquid, a cleaning liquid, a rinsing liquid, a storing liquid or a multi-purpose liquid which has two or more functions such as combinations of disinfecting, cleaning, rinsing and storing functions, each of which contains the stain-preventing treating composition of the present invention. When the multi-purpose liquid as described above is used in treating the contact lens in a conventional manner, the contact lens can be disinfected, cleaned, rinsed and stored while it is at the same time given the anti-staining or stain-preventing property, by a single liquid agent.

For instance, the contact lens is held in contact with the present treating composition in a liquid form (i.e., a treating solution) at a temperature of not lower than 80°C for not less than five minutes, preferably at a temperature of 80-110°C for 5-60 minutes, whereby the contact lens is subjected simultaneously to the stain-preventing treatment and the thermal disinfecting treatment. In this arrangement, a commercially available heating unit or device which is conventionally used in the thermal disinfecting treatment can be used.

The treating solution as described above may include an effective amount of disinfectant suitably selected from among any known disinfectants, whereby the contact lens is simultaneously subjected to the chemical disinfecting treatment concurrently with the stain-preventing treatment, by using this treating solution. The disinfectant to be included in the treating solution should not give an adverse influence on the stain-preventing effect of the present treating composition. In view of this, the disinfectant is suitably selected from among free compounds such as polyhexamethylene biguanide (PHMB), chlorhexidine and quaternary ammonium salts (such as benzalkonium chloride and polyquaternium-1), and salts of these free compounds. The concentration of the disinfectant to be included in the treating solution is suitably determined depending upon the kinds of the disinfectant and the desired disinfecting effect to be obtained. When PHMB (salt form) is used as the disinfectant, its concentration is adjusted to within a range of 0.1-5ppm, preferably 0.3-1ppm. For chlorhexidine (salt form) and quaternary ammonium salt, the concentrations are adjusted to within a range of 1-50ppm and 10-100ppm, respectively. By adding the disinfectants whose concentrations are adjusted to the respective suitable values, the treating solution exhibits an excellent disinfecting effect. The contact lens can be simultaneously subjected to the stain-preventing treatment and the chemical disinfecting treatment by immersing the contact lens in the thus prepared treating solution which contains the disinfectant as described above, at the room temperature for 1-24 hours, preferably for 2-6 hours.

In general, the contact lens is stored while it is immersed in a suitable liquid. Since the non-water-swellable contact lens is generally stored at a temperature around the room temperature, the treating solution of the present invention can be used as a storing liquid for storing the non-water-swellable contact lens. In view of the fact that the microorganisms or germs tend to proliferate on the water-swellable contact lens, the above-described disinfectant which also exhibits a preservative effect is added to the present treating solution, so that the present treating solution can be used as a storing liquid for storing the water-swellable contact lens.

In an attempt to improve the effect for removing the protein deposits and lipid deposits adhering to the contact lens, enzymes such as a protease and a lipase having functions of decomposing the proteins and the lipids, respectively, may be added to the present treating solution. In case that the contact lens is stained beyond the stain-preventing capability of the present treating composition, these enzymes are capable of removing the deposits from the contact lens, so as to effectively prevent accumulation of the deposits on the contact lens. Preferably, the protease is added to the present treating solution for removing the protein deposits. While any known protease may be added to the present treating solution, it is preferable to use a serine protease such as subtilisin or a thiol protease such as papain. The concentration of the protease to be added to the present treating composition is suitably determined depending upon the desired protein-removal effect to be obtained.

According to the present invention, the contact lens is simultaneously subjected to the stain-preventing treatment, the disinfecting treatment and the cleaning treatment in a single step, to thereby simplify the maintenance of the contact lens.

In using the treating solution that contains the present treating composition for preventing the staining of the contact lens as the multi-purpose liquid agent, it is preferable to add suitable additives thereto which are used in the conventional contact lens liquid agents. Any known additives such as a surface active agent, a chelating agent and a tonicity agent may be added to the present treating solution, provided that they are safe to the living body and that they do not give an adverse influence on the material of the contact lens. These additives are added to the treating solution in combination as needed in amounts that do not inhibit the stain-preventing effect exhibited by the effective component of 2-amino-2-methyl-1,3-propanediol or 2-amino-2-methyl-1-propanol in the present treating composition.

Described more specifically, a suitable surface active agent may be added to improve the effect of removing the lipid deposits such as the lipid in the eyes adhering to the contact lens, for example. Any known surface active agents such as an anionic surface active agent, an amphoteric surface active agent and a nonionic surface active agent may be used as long as they are safe to the living body and they do not give an adverse influence on the material of the contact lens. When the present treating solution that includes the stain-preventing composition is used to treat the water-swellable contact lens, it is preferable to employ the nonionic surface active agent for preventing adsorption thereof to the contact lens. Examples of the nonionic surface active agent include polyoxyethylene-polyoxypropylene block polymer, polyoxyethylene sorbitan fatty acid ester, and polyoxyethylene sorbitol fatty acid ester. While the concentration of the nonionic surface active agent used in the present treating liquid is suitably determined depending upon the desired cleaning effect to be obtained, it is generally in a range of 0.005-5 wt.%, preferably 0.02-1 wt. %, more preferably 0.05-0.5 wt.%.

As the chelating agent for removing the inorganic matters, ethylenediamine tetraacetic acid (EDTA), hydroxyethyl ethylenediamine triacetic acid (HEDTA), nitrilotriacetic acid or sodium salts thereof is employed, for instance. It is particularly preferable to employ EDTA and a sodium salt thereof. The concentration of the chelating agent included in the present treating liquid is generally in a range of 0.001-1 wt.%, preferably 0.01-0.2 wt.%, more preferably 0.01-0.1 wt.%. If the concentration of the chelating agent is lower than 0.001 wt.%, the contact lens may suffer from deposition of calcium which is derived from the tear fluid. The effect of removing the inorganic substances does not increase with an increase in the concentration of the chelating agent above 1 wt.%. On the other hand, the inclusion of the chelating agent exceeding 1 wt.% may even cause a trouble to the living body.

The present treating liquid which includes the above-described additives as needed has an osmotic pressure that is held within a physiologically permissible range. More specifically, the osmotic pressure of the treating liquid is adjusted to within a range of 200-600 mOsm, preferably 220-450 mOsm. For adjusting the osmotic pressure of the treating liquid in the above preferred ranges, any known tonicity agents may be used as long as they are safe to the living body. Generally, any one or any combination of sodium chloride, potassium chloride, sodium bicarbonate and glycerin may be used as the tonicity agent. The concentration of the tonicity agent in the treating liquid is determined so that the treating liquid has the intended osmotic pressure, while taking account of the amounts of the other additives.

In another aspect of the present invention, the above-described compound(s) included in the treating composition for preventing the staining of the contact lens, i.e., 2-amino-2-methyl-2,3-propanediol, 2-amino-2-methyl-1-propanol or salts thereof, is used as an effective component for stabilizing the protease. The compound is prepared into a liquid cleaning composition for cleaning a contact lens, and the contact lens is cleaned by using the liquid cleaning composition, to thereby provide an excellent protein removal effect to be exhibited by the protease which is stabilized by the above-described compound(s), in addition to an excellent stain-preventing effect as described above.

The protease which is included in the present liquid cleaning composition for a contact lens as one essential component has a function of removing the protein deposits adhering to the contact lens. While the amount of the protease included in the present liquid cleaning composition is suitably determined depending upon the desired protein removal effect to be obtained and kinds of the protease, the effective amount of the protease in the liquid cleaning composition is generally in a range of 0.005-10 w/v%, preferably 0.05-5 w/v%. The protease is generally classified, depending upon kinds of a residue in an active site thereof, into: serine protease; thiol protease; metal protease; and carboxyl protease. In particular, the serine protease is preferably used in the contact lens liquid cleaning composition of the present invention. This is because the serine protease does not require any cofactor, to thereby assure easy handling thereof. On the other hand, the thiol protease and carboxyl protease need to be activated, making the handling cumbersome.

The serine protease preferably used in the present invention includes a serine residue in its active site. Described in-detail, trypsin and chymotrypsin derived from an animal, and protease derived from bacteria, actinomycetes and molds are used. Various kinds of the protease which are derived from Bacillus are commercially available, such as "Bioprase" from NAGASE SEIKAGAKU KOGYO, K.K., "Clear Lens Pro", "Esperase" and "Durazym" from Novo Nordisk Bioindustry, Ltd., and "Alkali Protease GL-440" from Kyowa-Enzyme K.K.

The molecules of the serine protease which are derived from Bacillus have portions for bonding to calcium ion. With the calcium ion being bonded to these portions, the enzyme has a stable molecular structure. In view of this, when the serine protease which is derived from the Bacillus is used in the present liquid cleaning composition, the calcium ion is added for increasing the stability of the enzyme. Accordingly, the present liquid cleaning composition assures improved detergency with respect to the protein deposits.

The calcium ion is generally provided in the form of a calcium salt which generally has good water solubility, such as calcium chloride, calcium sulfate or calcium acetate. The calcium salt is added as the calcium ion to the present liquid cleaning composition generally with a concentration of not higher than 30 mM, preferably in a range of 3-20 mM. The enzyme stabilizing effect to be exhibited by the calcium ion does not significantly increase with an increase in the concentration exceeding the upper limit.

Since the protease as described above is generally unstable in an aqueous medium, a specific stabilizing component (solvent) needs to be added when the protease is provided in a liquid form. It is necessary to select the stabilizing component which effectively stabilizes the protease while at the same time, it assures a high degree of safety to the living body and it does not adversely influence the material of the contact lens. In the light of this, at least one compound selected from the group consisting of 2-amino-2-methyl-1,3-propanediol, 2-amino-2-methyl-1-propanol and salts thereof is used as the stabilizing component for stabilizing the protease. That is, in the present invention, the above-described at least one compound is added as the stabilizing component to the liquid cleaning composition for a contact lens, whereby the protease is effectively stabilized so as to improve the detergency with respect to the protein deposits.

The above-described stabilizing component of the present invention is included in the aqueous medium in an amount of at least 5 w/v%, to thereby stabilize the protease in the liquid cleaning composition. Preferably, the stabilizing component is included in the aqueous medium in an amount of not less than 10 w/v%. In particular, the enzyme is effectively stabilized by the stabilizing component if the stabilizing component is present in the aqueous medium with a relatively high concentration. For stabilizing the protease in a liquid form to a sufficient extent, it is preferable that the stabilizing component be included generally in an amount of 20-70 w/v%, preferably in an amount of 30-70 w/v%, more preferably in an amount of 45-65 w/v%. The stabilizing component may be used in combination with a water-soluble organic solvent such as glycerin or propylene glycol as needed.

As in the treating composition for preventing the staining of the contact lens as described above, the stabilizing component, i.e., 2-amino-2-methyl-1,3-propanediol or 2-amino-2-methyl-1-propanol, is provided in a free state or in a salt thereof such as hydrochloride. When the stabilizing component is provided in a free state, the aqueous solution thereof is alkaline. When the stabilizing component is provided in a salt thereof such as hydrochloride, the aqueous solution thereof is acidic. In order to keep the present liquid cleaning composition within an optimum pH range of 5.5-7.5, at least one acid or at least one alkali is suitably added to the composition. Examples of the acid and alkali are the same as described above with respect to the treating composition for preventing the staining of the contact lens.

To the present liquid cleaning composition for a contact lens which contains the specific stabilizing component and the protease as described above, there may be added as needed a surface active agent for improving an effect of removing the lipid deposits such as the lipid in the eye adhering to the contact lens. Any known surface active agents such as an anionic surface active agent, an amphoteric surface active agent and a nonionic surface active agent may be added as long as they assure a high degree of safety to the living body and that they do not adversely influence the material of the contact lens. When the present liquid cleaning composition is used to treat the water-swellable contact lens, it is preferable to employ the nonionic surface active agent for avoiding the adsorption thereof to the contact lens. Examples of the nonionic surface active agent include polyoxyethylene-polyoxypropylene block polymer, condensation product of polyoxyethylene or polyoxypropylene and ethylenediamine, glycerin fatty acid ester, polyoxyethylene glycerin fatty acid ester, sucrose alkyl ester, polyoxyethylene alkylamine, polyoxyethylene sorbitan fatty acid ester, and polyoxyethylene hardened castor oil. Particularly preferable is polyoxyethylene sorbitan fatty acid ester. While the concentration of the surface active agent included in the present liquid cleaning composition is suitably determined depending upon the desired detergency to be obtained, it is generally in a range of 0-10 w/v%, preferably 0.01-5 w/v%.

The contact lens may suffer from deposition of the calcium which is derived from the tear fluid, as well as the deposition of the protein and the lipid. In view of this, the present liquid cleaning composition may further include a metal chelating agent such as ethylenediamine tetraacetic acid (EDTA), hydroxyethyl ethylenediamine triacetic acid (HEDTA), nitrilotriacetic acid, or salts thereof such as sodium salts. The chelating agent is included in the present liquid cleaning composition such that the concentration in a dilution obtained by diluting the liquid cleaning composition to clean the contact lens is generally in a range of 0.001-1 w/v%, preferably 0.01-0.2 w/v%.

The liquid cleaning composition according to the present invention may further contain a preservative or a disinfectant with a suitable concentration determined depending upon the desired disinfecting or sterilizing effect to be obtained, for the purpose of improving its quality, e.g., for inhibition of proliferation of the microorganisms and sterilization during storage or use of the liquid cleaning composition. Such a preservative or a disinfectant is selected, for example, from among: sorbic acid, benzoic acid and salts thereof; biguanide such as polyhexamethylene biguanide (PHMB); and free compounds such as chlorhexidine or quaternary ammonium salt (such as benzalkonium chloride or polyquaternium-1) and salts thereof.

It is needless to say that various other additives used in the conventional contact lens treating agents may be added as needed to the present liquid cleaning composition, provided that they are safe to the living body and that they do not adversely influence the material of the contact lens. The additives are used in combination as needed, and added to the present liquid cleaning composition in amounts that do not inhibit the cleaning effect and the stabilizing effect of the present liquid cleaning composition.

The present liquid cleaning composition for a contact lens is obtained by adding a suitable aqueous medium to the mixture of the protease, the at least one stabilizing component selected from the group consisting of 2-amino-2-methyl-1,3-propanediol, 2-amino-2-methyl-1-propanol and salts thereof, and the additives as needed. As the aqueous medium, it is possible to use, in addition to water such as purified water and distilled water, known liquid agents for the contact lens such as a cleaning liquid, a storing liquid and a disinfecting liquid as long as they are aqueous solutions.

In cleaning the contact lenses which are classified into hard contact lenses and soft contact lenses, and water-swellable contact lenses and non-water-swellable contact lenses, by using the present' liquid cleaning composition prepared as described above, the liquid cleaning composition is initially diluted with a suitable aqueous diluting medium to a physiologically permissible extent. By thus diluting the liquid cleaning composition, the proportion of the stabilizing component included therein deviates from the range which assures the stabilization of the protease, whereby the protease included in the liquid cleaning composition is activated, so as to considerably increase the cleaning effect to be exhibited by the protease.

The liquid cleaning composition is uniformly mixed with, and dissolved in the aqueous diluting medium in an amount of 0.1-100 parts by volume, preferably in an amount of 0.5-5 parts by volume, per 100 parts by volume of the aqueous diluting medium. Accordingly, the obtained dilution has a physiologically permissible osmotic pressure, namely, in a range of 200-600 mOsm, preferably in a range of 250-400 mOsm, and a physiologically permissible pH, namely in a range of 5.5-8.0, preferably in a range of 6.5-7.5. The liquid cleaning composition is diluted so that the stabilizing component has a concentration of not higher than 3 w/v%, and that the protease has a concentration of 0.00001-1.0 w/v%.

The aqueous diluting medium for diluting the liquid cleaning composition is suitably selected from among tap water, purified water, distilled water, physiological salt solution or saline, conventional contact lens cleaning liquids (aqueous solutions containing a surface active agent), and conventional contact lens storing liquids (aqueous solutions containing sodium chloride), for instance. It is not appropriate to use the tap water as the aqueous diluting medium if it includes any impurities which reduce the desired effect to be exhibited by the protease included in the cleaning composition. Accordingly, it is preferable to use, as the aqueous diluting medium, purified water that is free from the impurities, a physiological salt solution, an aqueous solution containing a surface active agent, and an aqueous solution containing sodium chloride, for instance. It is particularly preferable to use commercially available contact lens cleaning liquids which are aqueous solutions containing a surface active agent. The conventionally used contact lens disinfecting liquids which are aqueous solutions containing a disinfectant are also used as the aqueous diluting medium, so that the contact lens can be disinfected concurrently with the protein deposits-removing treatment and the stain-preventing treatment.

According to the present method of cleaning a contact lens, the contact lens which was worn on the eye is immersed in the dilution obtained by diluting the liquid cleaning composition as described above with a suitable dilution, so that the deposits on the contact lens surface are removed (decomposed or separated). The contact lens is immersed in the dilution generally for a time period ranging from two hours to one night at room temperature. After the immersion, the contact lens is taken out of the dilution, and is rubbed with fingers by using the conventional contact lens cleaning liquid or storing liquid. Thereafter, the contact lens is rinsed with tap water. The cleaning operation at room temperature as described above is suitably effected on the non-water-swellable contact lenses. In this case, since the disinfectant is present in the dilution, which disinfectant is derived from the liquid cleaning composition or the aqueous diluting medium, the non-water swellable contact lenses can be disinfected concurrently with the cleaning operation.

When the water-swellable contact lenses are cleaned by the present liquid cleaning composition, the contact lenses are preferably subjected to the thermal disinfecting treatment at high temperature, in addition to the immersion of the contact lenses in the above-described dilution. More specifically, the contact lenses are disinfected at a temperature of not lower than 60°C for not less than five minutes, preferably at a temperature of not lower than 80°C for not less than five minutes. It is particularly preferable to boil the contact lenses. After the thermal disinfecting treatment as described above, the contact lenses are taken out of the dilution, rubbed with fingers, and then rinsed in the same manner as described above, whereby the contact lenses can be cleaned and disinfected.

More specifically described, one droplet of the present liquid cleaning composition is poured into a commercially available contact lens case, and then the lens case is filled with the physiological salt solution. After the worn contact lens is lightly rinsed with the physiological salt solution, it is accommodated in the lens case. Then, the lens case is installed on a commercially available contact lens boiling device (adapted to heat the contact lens in two different temperature ranges for cleaning and boiling·sterilizing the contact lens), so that the contact lens is boiled for sterilization. Thereafter, the contact lens is taken out of the lens case, and rinsed with the physiological salt solution before it is worn on the eye.

According to the present invention, the contact lens which was worn on the eye is immersed in the dilution obtained by diluting the liquid cleaning composition which includes the specific stabilizing component, so that the deposits such as the protein deposits adhering to the contact lens can be effectively removed from the contact lens. In addition, the contact lens is free from the problem of adhesion of the decomposed protein deposits back to the contact lens, which was encountered during treatment of the contact lens by using the conventional enzyme-containing treating liquid. Furthermore, the present cleaning method minimizes or prevents the staining of the contact lens, especially with the proteinaceous substance in the subsequent wearing of the contact lens.

In the above explanation, the dilution obtained by diluting the present liquid cleaning composition is used for cleaning the contact lens. However, the dilution may be also used as the storing liquid for storing the contact lens. Generally, the contact lens is stored while it is immersed in a suitable liquid. Since the non-water-swellable contact lens is usually stored at room temperature, the dilution prepared as described above can be used as the storing liquid. If the dilution includes a suitable disinfectant which also has a preservative effect, it can be used as the storing liquid for storing the water-swellable contact lens on which the microorganisms such as germs tend to proliferate.

Examples

To further clarify the concept of the present invention, some examples of the invention will be described. It is to be understood, however, that the present invention is not limited to the details of the illustrated examples, but may be embodied with various changes, modifications and improvements, which may occur to those skilled in the art without departing from the spirit of the present invention.

<Example 1>

---Test for confirming the stain-preventing effect---

Initially, there were prepared liquid specimens Nos. 1-8 which contain the treating composition for preventing the staining of the contact lens according to the present invention, such that the liquid specimens have the respective compositions as indicated in the following Table 1.

Namely, predetermined amounts of 2-amino-2-methyl-1,3-propanediol (AMPD) and 2-amino-2-methyl-1-propanol-HCl salt (AMP·HCl) as indicated in the Table 1 are respectively dissolved in 80mL of purified water, together with 0.5g of sodium chloride. The pH of the thus obtained solutions which give the liquid specimens Nos. 1, 3 and 4 was adjusted to 7.0 by using a 3N hydrochloric acid while the pH of the solutions which give the liquid specimens Nos. 2, 5 and 6 was adjusted to 7.0 by using a 3N sodium hydroxide. A measured 100 mL volume of each solution was used as the intended liquid specimen. The pH of the solutions which give the liquid specimens Nos. 7 and 8 was adjusted to 6.0 and 8.0, respectively, by using hydrochloric acid. Similarly, a measured 100 mL volume of each solution was used as the intended liquid specimen.

Each of the thus obtained liquid specimens Nos. 1-8 was measured of its pH by a glass electrode pH meter, and was also measured of its osmotic pressure by a freezing-point osmometer. The measured values are also indicated in the Table 1.

Next, there was prepared 1% lysozyme solution that contains lysozyme which was labelled with $^{125}$I as a radioactive tracer, in an amount indicating about 10000cpm per 100$\mu$g. Into a plurality of lens cases, there were respectively poured 0.2mL of the lysozyme solution and 1.8mL of the liquid specimens Nos. 1-8. A water-absorbable contact lens ("MENICON SOFT MA" available from Menicon, Co., Ltd.) was put into each of the lens cases, and was boiled at about 100°C for 15 minutes by using a contact lens boiling device ("Thermolizer" available from Tanica Denki, Co., Ltd.). After the boiling operation, the contact lens was taken out of the corresponding lens case, and was fully rinsed with the physiological salt solution. Thereafter, each contact lens was put into a vial for a gamma counter so as to measure the radioactivity (A cpm) by an auto well gamma counter. For comparison, there were measured the radioactivity (B cpm) of 100$\mu$g of lysozyme chloride and a background (BK cpm) of an empty vial. The amount of the lysozyme which adhered to each contact lens was calculated according to the following equation. The calculated value is also indicated in the Table 1.

$$\text{the amount } (\mu g) \text{ of lysozyme which adhered to the contact lens}$$

$$= (A(cpm) - B(cpm)) \times 100 - \div \{B(cpm) - BK(cpm)\}$$

In the same manner as described above, there were prepared liquid specimens Nos. 1-5 as comparative examples, so as to have the respective compositions as indicated in the following Table 2. After each of the liquid specimens according to the comparative examples was measured of its pH and osmotic pressure, the amount of the lysozyme which adhered to the contact lens was obtained in the same manner as described above with respect to the liquid specimens Nos. 1-8 according to the present invention. The results are also indicated in the Table 2.

**TABLE 1**

| | Preent invention | | | | | | | |
|---|---|---|---|---|---|---|---|---|
| | 1 | 2 | 3 | 4 | 5 | 6 | 7 | 8 |
| AMPD (g) | 0.73 | --- | 0.20 | 1.64 | --- | --- | 0.73 | 0.73 |
| AMP·HCl (g) | --- | 0.85 | --- | --- | 0.20 | 1.91 | --- | --- |
| hydrochloric acid | * | --- | * | * | --- | --- | * | * |
| sodium hydroxide | --- | * | --- | --- | * | * | --- | --- |
| pH | 6.99 | 7.00 | 6.98 | 6.98 | 6.99 | 7.00 | 6.00 | 8.04 |
| osmotic pressure (mOsm) | 285 | 284 | 288 | 288 | 285 | 282 | 295 | 279 |
| amount of lysozyme adhering to contact lens ($\mu$g) | 18.9 | 11.0 | 15.8 | 12.6 | 19.6 | 7.7 | 15.1 | 44.4 |
| *: suitable amounts | | | | | | | | |

**TABLE 2**

| | Comparative examples | | | | |
|---|---|---|---|---|---|
| | 1 | 2 | 3 | 4 | 5 |
| sodium chloride (g) | 0.50 | 0.50 | 0.50 | 0.50 | 0.50 |
| boric acid (g) | 0.80 | --- | --- | --- | --- |
| trisodium citrate (g) | --- | 1.32 | --- | --- | --- |
| disodium hydrogenphosphate dodecahydrate (g) | --- | --- | 1.70 | --- | --- |
| sodium tripolyphosphate (g) | --- | --- | --- | 1.00 | --- |
| 1,3-propanediol (g) | --- | --- | --- | --- | 0.95 |
| hydrochloric acid | --- | * | * | * | --- |
| sodium hydroxide | * | --- | --- | --- | * |
| pH | 7.20 | 7.08 | 7.04 | 7.02 | 7.07 |
| osmotic pressure (mOsm) | 287 | 286 | 289 | 292 | 292 |
| amount of lysozyme adhering to contact lens (μg) | 236.9 | 310.6 | 315.7 | 311.5 | 137.5 |
| *: suitable amounts | | | | | |

As is apparent from the results indicated in the above Tables 1 and 2, the amounts of the lysozyme adhering to the contact lenses were significantly small when the contact lenses were treated by the liquid specimens Nos. 1-8 which contain the treating composition for preventing the staining of the contact lens according to the present invention, as compared with those when the contact lenses were treated by the liquid specimens Nos. 1-5 of the comparative examples. Thus, it is clear that the present treating composition for preventing the staining of the contact lens assures an excellent stain-preventing effect.

Example 2

---Influence of the additives---

Liquid specimens Nos. 9-12 according to the present invention and a liquid specimen No. 6 as a comparative example were prepared in the same manner as in the above Example 1, so as to have the respective compositions as indicated in the following Table 3. In this Example 2, the liquid specimens include sodium edetate as a chelating agent, polyoxyethylene-polypropyleneglycol as a nonionic surface active agent, and polyhexamethylene biguanide·HCl salt as a preservative.

In the same manner as in the Example 1, the amounts of the lysozyme which adhered to the contact lenses were calculated. The results are also shown in the Table 3.

**TABLE 3**

| | Present invention | | | | Comparative example |
|---|---|---|---|---|---|
| | 9 | 10 | 11 | 12 | 6 |
| AMPD (g) | 0.73 | --- | --- | 0.73 | --- |
| AMP·HCl (g) | --- | 0.85 | 0.85 | --- | --- |
| boric acid (g) | --- | --- | --- | --- | 0.8 |
| sodium chloride (g) | 0.5 | 0.5 | 0.5 | 0.5 | 0.5 |
| hydrochloric acid | * | --- | --- | * | --- |
| sodium hydroxide | --- | * | * | --- | * |
| chelating agent†[1] (g) | --- | --- | --- | 0.02 | --- |

(continued)

| | | Present invention | | | | Comparative example |
|---|---|---|---|---|---|---|
| | | 9 | 10 | 11 | 12 | 6 |
| nonionic surface active agent | A†[2] (g) | 0.1 | --- | --- | 0.1 | --- |
| | B†[3] (g) | --- | 0.1 | --- | --- | --- |
| disinfectant†[4] (mg) | | --- | --- | 0.03 | 0.03 | --- |
| pH | | 6.99 | 7.00 | 7.00 | 7.00 | 7.20 |
| osmotic pressure (mOsm) | | 286 | 283 | 282 | 287 | 287 |
| amount of lysozyme adhering to contact lens (μg) | | 7.2 | 7.4 | 41.7 | 8.7 | 214.0 |
| *: suitable amounts<br>†[1]: sodium edetate<br>†[2]: polyoxyethylene (196) polyoxypropylene (67) glycol<br>†[3]: polyoxyethylene (20) sorbitan monooleate<br>†[4]: polyhexamethylene biguanide·HCl salt | | | | | | |

It will be understood that the present treating composition for preventing the staining of the contact lens exhibited an excellent stain-preventing effect even when it is used in combination with the additives such as the chelating agent, surface active agent and preservative.

Example 3

- --Stain-preventing treatment in combination with chemical disinfecting treatment--

Various liquid specimens were prepared in the same manner as in the above Example 1, so as to have the respective compositions as indicated in the following Table 4. In this Example 3, the liquid specimens include, as a preservative or disinfectant, polyhexamethylene biguanide·HCl salt or chlorhexidine gluconate, so that the chemical disinfecting treatment is simultaneously effected.

Initially, there was prepared 1% lysozyme solution including lysozyme that was labelled with $^{125}$I as a radioactive tracer in an amount indicating 60000cpm per 100μg. 0.2mL of the thus prepared lysozyme solution and 1.8mL of the liquid specimens Nos. 13-15 according to the present invention and the liquid specimen No. 7 according to the comparative example were respectively poured into contact. lens cases. A water-absorbable contact lens ("MENICON SOFT MA" available from Menicon Co., Ltd.) was put into each of the lens cases, and kept at room temperature of about 25°C for 16 hours. Thereafter, the contact lens was taken out of the corresponding lens case, and was measured of the amount of the lysozyme which adhered thereto, in the same manner as in the above Example 1. The results are also shown in the Table 4.

In the meantime, there was prepared 1% lysozyme solution including lysozyme that was labelled with $^{125}$I as a radioactive tracer in an amount indicating 17500cpm per 100μg. 0.2mL of the thus prepared lysozyme solution and 1.8mL of the liquid specimens Nos. 13-15 according to the present invention and the liquid specimen No. 7 according to the comparative example were respectively poured into contact lens cases. A water-absorbable contact lens ("SUREVUE" available from Johnson & Johnson Vision Products Inc.) was put into each of the lens cases, and kept at room temperature of about 25°C for 16 hours. Thereafter, the contact lens was taken out of the corresponding lens case, and was measured of the amount of the lysozyme which adhered thereto, in the same manner as in the above Example 1. The results are also shown in the Table 4.

The disinfecting or antimicrobial effect of the liquid specimens prepared as described above was examined in the following manner. Initially, the following bacteria and fungi were incubated in a suitable manner: S.a. (Staphylococcus aureus ATCC 6538); P.a. (Pseudomonas aeruginosa ATCC 9027); E.c. (Eschrichia coli ATCC 8739); S.m. (Serratia marcescens ATCC 13880); C.a. (Candida albicans ATCC 10231); and A.f. (Aspergillus fumigatus ATCC 10894). The incubated bacteria and fungi were suspended in a physiological salt solution, so as to respectively provide bacterium liquids and fungus liquids, each of which contains the corresponding bacterium or fungus in an amount of $10^8$-$10^9$ cfu/mL. 9.9mL of the liquid specimens Nos. 13-15 according to the present invention and the liquid specimen No. 7 according to the comparative example were put into sterilized test tubes, respectively. To each of the test tubes, there was added 0.1mL of each bacterium or fungus liquid prepared as described above, so as to provide a bacterium or fungus suspension having a concentration of $10^6$-$10^7$ cfu/mL. After the thus obtained suspension was kept at 23°C for four hours, there

# EP 0 888 780 B2

was calculated a viable cell count per 1mL of the suspension according to an agar plate dilution method.

Then, a ratio of reduction (Log Reduction) of each bacterium or fungus was calculated in logarithm according to the following equation, based on the viable cell count immediately after the preparation of the bacterium or fungus suspension and the viable cell count four hours after the preparation of the bacterium or fungus suspension.

$$\text{Reduction amount} =$$

$$\log\{(\text{the viable cell count per 1 mL of each bacterium or}$$

$$\text{fungus suspension immediately after the preparation}) -$$

$$(\text{the viable cell count per 1 mL of each bacterium or}$$

$$\text{fungus suspension after the treatment by each specimen}$$

$$\text{liquid of the contact lens treating composition})$$

**TABLE 4**

| | | Present invention | | | Comparative example |
|---|---|---|---|---|---|
| | | 13 | 14 | 15 | 7 |
| AMPD (g) | | 0.73 | --- | 0.73 | --- |
| AMP·HCl (g) | | --- | 0.85 | --- | --- |
| boric acid (g) | | --- | --- | --- | 0.8 |
| sodium chloride (g) | | 0.5 | 0.5 | 0.5 | 0.5 |
| hydrochloric acid | | * | --- | * | --- |
| sodium hydroxide | | --- | * | --- | * |
| chelating agent†[1] (g) | | --- | --- | 0.02 | --- |
| nonionic surface active agent†[2] (g) | | --- | --- | 0.2 | --- |
| disinfectant | A†[3] (mg) | 0.1 | --- | 0.1 | 0.1 |
| | B†[4] (mg) | --- | 5 | --- | --- |
| pH | | 6.99 | 6.97 | 6.98 | 7.00 |
| osmotic pressure (mOsm) | | 286 | 282 | 289 | 289 |
| amount of lysozyme adhering to contact lens (μg) | MENICON SOFT MA | 11.5 | 12.2 | 10.9 | 16.4 |
| | SUREVUE | 485 | 412 | 493 | 976 |

13

(continued)

| | | Present invention | | | Comparative example |
|---|---|---|---|---|---|
| | | 13 | 14 | 15 | 7 |
| reduction amount of bacteria or fungi (log) | S.m. | 3.04 | 4.94 | 2.27 | 2.06 |
| | P.a. | 4.02 | 6.02 | 3.94 | 4.08 |
| | E.c. | 5.34 | 5.96 | 4.94 | 4.26 |
| | S.a. | 1.76 | 1.50 | 2.02 | 1.83 |
| | C.a. | 0 | 2.18 | 0 | 0 |
| | A.f. | 0.11 | 1.47 | 0 | 0 |

*: suitable amounts
[1]: sodium edetate
[2]: polyoxyethylene (196) polyoxypropylene (67) glycol
[3]: polyhexamethylene biguanide·HCl
[4]: chlorhexidine gluconate

It will be understood from the results as shown in the Table 4 that the antimicrobial effect exhibited by the disinfectant did not deteriorate, but improved owing to the present treating composition for preventing the staining of the contact lens.

Example 5

---Effect for a hard contact lens---

In this Example 5, there were used the liquid specimens Nos. 1 and 2 according to the present invention and the liquid specimen No. 1 according to the comparative example prepared in the above Example 1, and the liquid specimen No. 15 according to the present invention prepared in the above Example 4.

Initially, 1% lysozyme solution was prepared which contains lysozyme that is labelled with $^{125}$I as a radioactive tracer in an amount indicating about 60000cpm per 100$\mu$g. 0.2mL of the thus prepared lysozyme solution and 1.8mL of the liquid specimens Nos. 1, 2 and 15 according to the present invention and the liquid specimen No. 1 according to the comparative example were put into contact lens cases, respectively. A hard contact lens ("MENICON EX" available from Menicon Co., Ltd.) was put into each of the lens cases, and kept at room temperature of about 25°C for 16 hours. Thereafter, each of the contact lenses was taken out of the corresponding lens case, and measured of the amount of the lysozyme which adhered thereto, in the same manner as in the above Example 1. The results are shown in the Table 5.

**TABLE 5**

| | Present invention | | | Comparative example |
|---|---|---|---|---|
| | 1 | 2 | 3 | 1 |
| amount of lysozyme adhering to contact lens ($\mu$g) | 8.9 | 11.0 | 6.0 | 16.7 |

As is apparent from the above results, the present treating composition for preventing the staining of the contact lens exhibited a significantly high degree of stain-preventing effect with respect to the hard contact lens.

It will be understood from the above description that the present treating composition for the contact lens minimizes or prevents the staining of the contact lens, especially with the proteinaceous substance.

When the present treating composition for preventing the staining of the contact lens includes suitable additives, the contact lens can be effectively subjected to the thermal disinfecting treatment, chemical disinfecting treatment, cleaning treatment and other necessary treatments concurrently with the stain-preventing treatment, without adversely influencing the stain-preventing effect and the antimicrobial effect.

EP 0 888 780 B2

Example 6

---Preparation of the liquid cleaning composition---

Various liquid cleaning composition specimens were prepared in the following manner. Initially, 2-amino-2-methyl-1,3-propanediol (AMPD) or 2-amino-2-methyl-1-propanol·HCl salt (AMP·HCl), and protease derived from Bacillus ("Esperase" available from Novo Nordisk Bioindustry Ltd.) were mixed in the respective amounts as indicated in the following Tables 6 and 7. Each of the mixtures was prepared into a 100ml volume of liquid cleaning composition by using purified water, the pH of which was adjusted to about 7.0 by adding hydrochloric acid or sodium hydroxide. Thus, the intended liquid cleaning composition specimens were obtained.

Subsequently, the enzyme activity was measured for each of the liquid cleaning composition specimens prepared as described above, in the manner described below. The enzyme activity was measured after each specimen was stored at 25°C for 14 days and after each specimen was stored at 40°C for 7 days. Then, the residual enzyme activity was calculated according to the following equation for each of the specimens. The results are also shown in the Tables 6 and 7.

---Measurement of the activity of the protease---

Initially, each specimen of the liquid cleaning composition was diluted with purified water at a suitable dilution ratio "D". To 1 ml of each diluted specimen of the liquid cleaning composition, 5ml of 0.6% casein solution (pH 7.0, 0.05M sodium monohydrogenphosphate aqueous solution) heated to 37°C was added. After the thus obtained mixture was kept at 37°C for ten minutes, there was added 5ml of a precipitating reagent (a mixed solution comprising 0.11M trichloroacetic acid, 0.22M sodium acetate and 0.33M acetic acid), whereby undecomposed protein was precipitated. Then, the mixture was subjected to filtration, and the filtrate was measured of its absorption A at 275nm. For comparison, each specimen of the liquid cleaning composition was diluted with purified water (at the dilution "D"). To 1ml of each diluted specimen of the liquid cleaning composition, 5ml of the above-described precipitating reagent was added. Further, the above-described casein solution was added, so that the protein was precipitated. The mixture was then subjected to filtration, and the obtained filtrate was measured of its absorption $A_0$ at 275nm.

The enzyme activity is evaluated as lu when the enzyme activity is capable of producing a non-protein substance showing an absorption amount at 275nm of $1 \times 10^{-6}$ of tyrosine per one minute. In the following equation, As represents an absorption of 50mg/ml of tyrosine at 275nm. In this example, As is equal to 391.

$$\text{Activity of protease (m/g)}$$

$$\fallingdotseq [(A - A_0)/As] \times 50 \times (11/10) \times D$$

$$\text{Residual enzyme activity (\%)}$$

$$= (\text{Activity of protease after the storage/}$$

$$\text{Activity of protease upon preparation}) \times 100$$

**TABLE 6**

| specimen No. | Esperase (g) | AMPD (g) | hydrochloric acid | residual enzyme activity (%) | |
| --- | --- | --- | --- | --- | --- |
| | | | | at 25°C after 14 days | at 40°C, after 7 days |
| 1 | 1.0 | 0 | --- | 18.2 | 0 |
| 2 | 1.0 | 10 | * | 33.6 | 0.3 |
| 3 | 1.0 | 20 | * | 48.8 | 5.4 |
| 4 | 1.0 | 25 | * | 79.2 | 8.9 |
| 5 | 1.0 | 30 | * | 85.7 | 25.8 |

(continued)

| specimen No. | Esperase (g) | AMPD (g) | hydrochloric acid | residual enzyme activity (%) | |
|---|---|---|---|---|---|
| | | | | at 25°C after 14 days | at 40°C, after 7 days |
| 6 | 1.0 | 35 | * | 100.4 | 50.3 |
| 7 | 1.0 | 40 | * | 100 | 76.1 |
| 8 | 1.0 | 45 | * | 100 | 85.7 |
| 9 | 1.0 | 50 | * | 100 | 91.0 |
| 10 | 1.0 | 55 | * | 100 | 96.8 |
| 11 | 1.0 | 60 | * | 100 | 100 |
| *: suitable amounts | | | | | |

**TABLE 7**

| specimen No. | Esperase (g) | AMP· HCl (g) | NaOH | residual enzyme activity (%) | |
|---|---|---|---|---|---|
| | | | | at 25°C after 14 days | at 40°C, after 7 days |
| 12 | 1.0 | 10 | * | 27.9 | 4.6 |
| 13 | 1.0 | 20 | * | 48.5 | 8.0 |
| 14 | 1.0 | 25 | * | 75.2 | 17.6 |
| 15 | 1.0 | 30 | * | 89.2 | 40.5 |
| 16 | 1.0 | 35 | * | 98.9 | 68.6 |
| 17 | 1.0 | 40 | * | 100 | 89.6 |
| 18 | 1.0 | 45 | * | 100 | 97.0 |
| 19 | 1.0 | 50 | * | 100 | 100 |
| 20 | 1.0 | 55 | * | 100 | 100 |
| 21 | 1.0 | 60 | * | 100 | 100 |
| 22 | 1.0 | 65 | * | 100 | 100 |
| 23 | 1.0 | 70 | * | 100 | 100 |
| *: suitable amounts | | | | | |

It will be understood from the above results that the protease (Esperase) was effectively stabilized by addition of suitable amounts of AMPD or AMP•HCl. In particular, the addition of AMPD or AMP•HCl in an amount of 20-70 w/v% is significantly effective to stabilize the protease. For stabilizing the protease to a sufficient extent, AMPD or AMP•HCl is added preferably in an amount of 30-70 w/v%, more preferably in an amount of 45-65 w/v%.

For comparison of the protease stability provided by AMPD or AMP·HCl as the stabilizing component according to the present invention and the protease stability provided by the known stabilizing component such as trometamol, glycerin or propylene glycol, there were prepared specimens Nos. 24-25 of the liquid cleaning composition according to the present invention and specimens Nos. 26-28 of the liquid cleaning composition as comparative examples, in the following manner. To the protease ("Clear Lens Pro 2.5MG" available from Novo Nordisk Bioindustry Ltd.), 2.5M of AMPD or AMP·HCl according to the present invention was added. Similarly, 2.5M of the known trometamol, glycerin or propylene glycol was added to the protease. The thus obtained mixtures were prepared into a 100ml volume of liquid cleaning compositions by using purified water, the pH of which was adjusted to 7.0 by hydrochloric acid or sodium hydroxide, so as to provide the intended specimens. The thus obtained specimens of the liquid cleaning composition were stored at 60°C for one day, or stored at 50°C for one week. For each of the specimens, the residual enzyme activity was calculated. The results are shown in the Table 8. It will be apparent from the results of the Table 8 that the specimens Nos. 24-25 of the liquid cleaning composition according to the present invention wherein AMPD or AMP•HCl is employed as the stabilizing component for stabilizing the protease exhibited a higher degree of enzyme stability than the specimens Nos. 26-28 of the liquid cleaning composition according to the comparative examples wherein the known stabilizing component such as trometamol, glycerin or propylene glycol is employed for stabilizing the protease.

**TABLE 8**

| components | | Present invention | | comparative examples | | |
|---|---|---|---|---|---|---|
| | | specimen No. | | | | |
| | | 24 | 25 | 26 | 27 | 28 |
| protease (g) | | 1.0 | 1.0 | 1.0 | 1.0 | 1.0 |
| AMPD (g) | | 16.3 | --- | --- | --- | --- |
| AMP·HCl (g) | | --- | 31.4 | --- | --- | --- |
| trometamol (g) | | --- | --- | 30.3 | --- | --- |
| glycerin (g) | | --- | --- | --- | 23.0 | --- |
| propylene glycol (g) | | --- | --- | --- | --- | 19.0 |
| calcium chloride dihydrate | | 0.06 | 0.06 | 0.06 | 0.06 | 0.06 |
| hydrochloric acid | | * | --- | * | --- | --- |
| sodium hydroxide | | --- | * | --- | * | * |
| residual enzyme activity (%) | at 60°C, after 1 day | 104.4 | 96.0 | 79.2 | 68.3 | 33.7 |
| | at 50°C, after 1 week | 80.3 | 90.4 | 28.2 | 32.1 | 16.5 |
| *: suitable amounts | | | | | | |

A test for confirming the stain-preventing effect was conducted for the above specimens Nos. 24 and 25 according to the present invention and the above specimens Nos. 27 and 28 according to the comparative examples, so as to evaluate the stain-preventing effect of each of the specimens of the liquid cleaning composition. Initially, two droplets of the liquid cleaning composition specimens Nos. 24, 25, 27 and 28 were poured into the respective contact lens cases, and diluted with 1.8ml of a commercially available storing liquid ("CLEAN BOTTLE SOAK" available from Menicon Co., Ltd.). Then, 0.2ml of 1% lysozyme solution was added as a proteinaceous substance to the dilution in each lens case. Subsequently, a water-absorbable contact lens ("MENICON SOFT MA" available from Menicon Co., Ltd.) was immersed in the dilution containing the proteinaceous substance, and was boiled for sterilization by heating the contact lens at two different temperatures (at 50°C for one hour and at 100°C for ten minutes) using a contact lens boil-sterilizer ("Thermolizer" available from Menicon Co., Ltd.). Thereafter, each contact lens was taken out of the corresponding lens case, and rinsed with a physiological salt solution by rubbing.

After the above-described treatment was repeated three times, the water-swellable contact lens was measured of the amount of the protein which adhered thereto by using a fluorophotometer. The results are indicated in the following Table 9. It will be apparent from the results that the amounts of the protein which adhered to the contact lenses were considerably large when the contact lenses were treated by the liquid cleaning composition specimens wherein the known stabilizing component such as glycerin or propylene glycol was employed for stabilizing the protease. On the contrary, in the contact lenses which were treated by the liquid cleaning composition specimens which employ AMPD or AMP·HCl as the stabilizing component, the amounts of the protein which adhered thereto were 1/2 to 1/10 of that in the contact lenses which were treated by the specimens according to the comparative examples.

**TABLE 9**

| | Present invention | | Comparative examples | |
|---|---|---|---|---|
| specimen No. | 24 | 25 | 27 | 28 |
| amount of protein adhering to contact lens (intensity) | 340 | 865 | 2005 | 1943 |

As is apparent from the above explanation, the present liquid cleaning composition for preventing the staining of a contact lens includes, as the stabilizing component for stabilizing the protease, 2-amino-2-methyl-1,3-propanediol, 2-amino-2-methyl-1-propanol or salts thereof, so that the protease is stabilized to a considerably high extent. Accordingly, the present liquid cleaning composition is capable of exhibiting an excellent enzyme activity even after a long period of storage. Further, the contact lens treated by the present liquid cleaning composition is effectively prevented from being stained, especially with the proteinaceous substance during cleaning and wearing thereof. In essence, the present liquid cleaning composition assures sufficiently high enzyme stability and stain-preventing effect at the same time.

According to the present method of cleaning a contact lens by using the liquid cleaning composition having the excellent characteristics as described above, the contact lens can be sufficiently cleaned and effectively prevented from being stained, by simply immersing the contact lens in a dilution obtained by diluting the present liquid cleaning composition with a suitable aqueous diluting medium.

Industrial Applicability

It will be understood from the above description that the present invention is utilized in preventing the contact lens from being stained, especially with the proteinaceous substance. Further, the present invention is utilized to effectively stabilize the protease, and improve the protein removal effect exhibited by the protease, in the liquid cleaning composition which contains the protease capable of decomposing and removing the proteinaceous substance of the contact lens.

**Claims**

1. Use of a composition to treat a contact lens, which composition includes at least one compound selected from the group consisting of 2-amino-2-methyl-1,3-propanediol, 2-amino-2-methyl-1-propanol, and salts thereof, as a component effective for preventing said contact lens from being stained, wherein said at least one compound is included in said treating composition in an amount of 0.01 - 3 wt%.

2. A composition for cleaning a contact lens which includes, in an aqueous medium, an effective amount of protease and not less than 5 w/v% of at least one compound as a stabilising component for stabilising said protease, said at least one compound being selected from the group consisting of 2-amino-2-methyl-1,3-propanediol, 2-amino-2-methyl-1-propanol, and salts thereof, and being a component effective for preventing said contact lens from being stained.

3. A composition according to claim 2, wherein the pH is adjusted to a value within a range of 5.5 - 7.5.

4. A composition according to claim 2 or 3, wherein said effective amount of protease is in a range of 0.005 - 10 w/v%.

5. A method of treating a contact lens for giving said contact lens an anti-staining property, said method comprising the steps of:

   (i) preparing a treating solution which includes 0.01 - 3 wt% of at least one compound selected from the group consisting of 2-amino-2-methyl-1,3-propanediol, 2-amino-2-methyl-1-propanol, and salts thereof, as a component effective for preventing said contact lens from being stained, and
   (ii) contacting said contact lens with said treating solution.

6. A method according to claim 5, wherein said contact lens is held in contact with said treating solution at a temperature of not lower than 80°C for not less than 5 minutes for simultaneously disinfecting said contact lens.

7. A method according to claim 5, wherein said treating solution further includes an effective amount of disinfectant, and said contact lens is held in contact with said treating solution at room temperature for simultaneously disinfecting said contact lens.

8. A method according to claim 5 or 6, wherein said treating solution further includes an effective amount of protease, and said contact lens is held in contact with said treating solution for simultaneously cleaning said contact lens.

9. A method according to claim 5, wherein said treating solution further includes effective amounts of disinfectant and protease, and said contact lens is held in contact with said treating solution at room temperature for simultaneously disinfecting and cleaning said contact lens.

10. A method according to claim 5, for cleaning the contact lens, wherein said step (i) has the steps of:

   preparing a first composition which includes, in an aqueous medium, an effective amount of protease and not less than 5 w/v% of said at least one compound as a stabilising component for stabilising said protease; diluting said first composition with an aqueous diluting medium so as to provide a dilution; and said step (ii) is:

immersing said contact lens which has been worn on a human eye in said dilution.

11. A method according to claim 10, further comprising the step of boiling said contact lens immersed in said dilution, for at least five minutes.

12. A method according to claim 10, wherein said dilution further includes an effective amount of disinfectant, and said contact lens is immersed in said dilution at room temperature for also disinfecting said contact lens.

13. A method according to any one of claims 10 to 12, wherein said at least one compound is included in an amount of 0:1 - 100 parts by volume per 100 parts by volume of said aqueous medium.

**Patentansprüche**

1. Verwendung einer Zusammensetzung zur Behandlung einer Kontaktlinse, die zumindest eine Verbindung, ausgewählt aus der aus 2-Amino-2-methyl-1,3-propandiol, 2-Amino-2-methyl-1-propanol und Salzen davon bestehenden Gruppe, als wirksame Komponente zur Verhinderung von Verschmutzung der Kontaktlinse enthält, worin die zumindest eine Verbindung in der Behandlungszusammensetzung in einer Menge von 0,01 bis 3 Gew.-% enthalten ist.

2. Zusammensetzung zur Reinigung einer Kontaktlinse, die in einem wässrigen Medium eine wirksame Menge an Protease und nicht weniger als 5 % (Gew.Nol.) der zumindest einen Verbindung als stabilisierende Komponente zur Stabilisierung der Protease enthält, wobei die zumindest eine Verbindung aus der aus 2-Amino-2-methyl-1,3-propandiol, 2-Amino-2-methyl-1-propanol und Salzen davon bestehenden Gruppe ausgewählt ist und eine Komponente ist, die zur Verhinderung von Verschmutzung der Kontaktlinse wirksam ist.

3. Zusammensetzung nach Anspruch 2, worin der pH auf einen Wert im Bereich 5,5 bis 7,5 eingestellt ist.

4. Zusammensetzung nach Anspruch 2 oder 3, worin die wirksame Menge an Protease in einem Bereich von 0,005 bis 10 % (Gew./Vol.) liegt.

5. Verfahren zur Behandlung einer Kontaktlinse, um der Kontaktlinse verschmutzungshemmende Eigenschaften zu verleihen, wobei das Verfahren folgende Schritte umfasst:

(i) Herstellung einer Behandlungslösung, die 0,01 bis 3 Gew.-% zumindest einer Verbindung, ausgewählt aus der aus 2-Amino-2-methyl-1,3-propandiol, 2-Amino-2-methyl-1-propanol und Salzen davon bestehenden Gruppe, als wirksame Komponente zur Verhinderung von Verschmutzung der Kontaktlinse enthält; und
(ii) Kontaktieren der Kontaktlinse mit der Behandlungslösung.

6. Verfahren nach Anspruch 5, worin die Kontaktlinse nicht weniger als 5 min lang bei einer Temperatur von nicht weniger als 80 °C mit der Behandlungslösung in Kontakt gehalten wird, um die Kontaktlinse gleichzeitig zu desinfizieren.

7. Verfahren nach Anspruch 5, worin die Behandlungslösung weiters eine wirksame Menge eines Desinfektionsmittels enthält und die Kontaktlinse bei Raumtemperatur mit der Behandlungslösung in Kontakt gehalten wird, um die Kontaktlinse gleichzeitig zu desinfizieren.

8. Verfahren nach Anspruch 5 oder 6, worin die Behandlungslösung weiters eine wirksame Menge an Protease enthält und die Kontaktlinse mit der Behandlungslösung in Kontakt gehalten wird, um die Kontaktlinse gleichzeitig zu reinigen.

9. Verfahren nach Anspruch 5, worin die Behandlungslösung weiters eine wirksame Menge an Desinfektionsmittel und an Protease enthält und die Kontaktlinse bei Raumtemperatur mit der Behandlungslösung in Kontakt gehalten wird, um die Kontaktlinse gleichzeitig zu desinfizieren und zu reinigen.

10. Verfahren nach Anspruch 5 zur Reinigung der Kontaktlinse, worin Schritt (i) folgende Schritte umfasst:

Herstellung einer ersten Zusammensetzung, die in einem wässrigen Medium eine wirksame Menge an Protease und nicht weniger als 5 % (Gew./Vol.) der zumindest einen Verbindung als stabilisierende Komponente zum Stabilisieren der Protease enthält; und

Verdünnen der ersten Zusammensetzung mit einem wässrigen Verdünnungsmedium, um eine Verdünnung bereitzustellen;

und Schritt (ii) im Eintauchen der Kontaktlinse, die an einem menschlichen Auge getragen worden ist, in die Verdünnung besteht.

11. Verfahren nach Anspruch 10, das weiters den Schritt des Kochens der in der Verdünnung eingetauchten Kontaktlinse für zumindest 5 min umfasst.

12. Verfahren nach Anspruch 10, worin die Verdünnung weiters eine wirksame Menge an Desinfektionsmittel umfasst und die Kontaktlinse bei Raumtemperatur in die Verdünnung eingetaucht wird, um die Kontaktlinse auch zu desinfizieren.

13. Verfahren nach einem der Ansprüche 10 bis 12, worin die zumindest eine Verbindung in einer Menge von 0,1 bis 100 Volumsteilen pro 100 Volumsteile des wässrigen Mediums enthalten ist.

**Revendications**

1. Utilisation d'une composition pour traiter une lentille de contact, ladite composition comprend au moins un composé sélectionné à partir du groupe constitué de 2-amino-2-méthyl-1,3-propanediol, 2-amino-2-méthyl-1-propanol, et des sels de ceux-ci, comme composant efficace pour empêcher que ladite lentille de contact ne soit tâchée, dans laquelle ledit au moins un composé est compris dans ladite composition de traitement en une quantité de 0,01 à 3 % en poids.

2. Composition pour nettoyer une lentille de contact, qui comprend, dans un milieu aqueux, une quantité efficace de protéase et pas moins de 5 % en poids dudit au moins un composé en tant que composant stabilisant pour stabiliser ladite protéase, ledit au moins un composé étant sélectionné à partir du groupe constitué de 2-amino-2-méthyl-1-3-propanediol, 2-amino-2-méthyl-1-propanol, et des sels de ceux-ci, et étant un composant efficace pour empêcher que ladite lentille de contact ne soit tâchée.

3. Composition selon la revendication 2, dans laquelle le pH est ajusté à une valeur dans une plage de 5,5 à 7,5.

4. Composition selon la revendication 2 ou 3, dans laquelle ladite quantité efficace de protéase est dans une plage de 0,005 à 10 % en poids.

5. Procédé de traitement d'une lentille de contact pour donner à ladite lentille de contact une propriété antitâche, ledit procédé comprenant les étapes de:

(i) préparer une solution de traitement qui comprend 0,01 à 3% en poids d'au moins un composé sélectionné à partir du groupe constitué de 2-amino-2-méthyl-1,3-propanediol, 2-amino-2-méthyl-1-propanol, et des sels de ceux-ci, comme composant efficace pour empêcher que ladite lentille de contact ne soit tâchée; et
(ii) mettre en contact ladite lentille de contact avec ladite solution de traitement.

6. Procédé selon la revendication 5, dans lequel ladite lentille de contact est maintenue en contact avec ladite solution de traitement à une température non inférieure à 80°C pendant au moins 5 minutes pour désinfecter simultanément ladite lentille de contact.

7. Procédé selon la revendication 5, dans lequel ladite solution de traitement comprend en outre une quantité efficace de désinfectant, et ladite lentille de contact est maintenue en contact avec ladite solution de traitement à température ambiante pour désinfecter simultanément ladite lentille de contact.

8. Procédé selon la revendication 5 ou 6, dans lequel ladite solution de traitement comprend en outre une quantité efficace de protéase, et ladite lentille de contact est maintenue en contact avec ladite solution de traitement pour nettoyer simultanément ladite lentille de contact.

9. Procédé selon la revendication 5, dans lequel ladite solution de traitement comprend en outre des quantités efficaces de désinfectant et de protéase, et ladite lentille de contact est maintenue en contact avec ladite solution de traitement à température ambiante pour désinfecter et nettoyer simultanément ladite lentille de contact.

10. Procédé selon la revendication 5, pour nettoyer la lentille de contact, dans lequel ladite étape (i) comprend les étapes de:

préparer une première composition qui comprend, dans un milieu aqueux, une quantité efficace de protéase et pas moins de 5 % en poids dudit au moins un composé comme composant stabilisant pour stabiliser ladite protéase;
diluer ladite première composition avec un milieu de dilution aqueux de manière à fournir une dilution; et ladite étape (ii) consiste à:

immerger ladite lentille de contact qui a été portée sur un oeil humain dans ladite dilution.

11. Procédé selon la revendication 10, comprenant en outre l'étape consistant à faire bouillir ladite lentille de contact immergée dans ladite dilution, pendant au moins cinq minutes.

12. Procédé selon la revendication 10, dans lequel ladite dilution comprend en outre une quantité efficace de désinfectant, et ladite lentille de contact est immergée dans ladite dilution à température ambiante pour désinfecter également ladite lentille de contact.

13. Procédé selon l'une quelconque des revendications 10 à 12, dans lequel ledit au moins un composé est compris dans une quantité de 0,1 à 100 parties en volume pour 100 parties en volume dudit milieu aqueux.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2168224 A
- JP 4093919 A
- JP 4143718 A
- JP 4161921 A